# EUROPEAN PATENT APPLICATION

(11) **EP 2 281 531 A1**
(43) Date of publication of application: **09.02.2011**
(21) Application number: 10250291.1
(22) Date of filing: 18.02.2010
(51) Int. Cl.: A61F 2/08

(54) **Method for repairing a rotator cuff**

(30) Priority: 19.02.2009 US 153676 P; 27.01.2010 US 694831
(62) Divisional of application: 10015033.3
(71) Applicant: Tyco Healthcare Group LP, North Haven, CT 06473 (US)
(72) Inventor: Scarborough, Nelson, Andover, MA 01810 (US)
(74) Representative: Soames, Candida Jane

(57) **Abstract**

A method of repairing a rotator cuff is disclosed. The method includes accessing a surgical site including a humerus and a rotator cuff tendon, debriding the rotator cuff tendon and creating a defect in the humerus. The method further includes providing a bone-tendon assembly including at least one graft tendon and at least one bone segment, attaching the at least one bone segment of the bone-tendon assembly within the bone defect such that the at least one graft tendon extends from the humerus and attaching the at least one graft tendon to the rotator cuff tendon.

## Description

### CROSS-REFERENCE TO RELATED APPLICATIONS

This application claims the benefit of and priority to U.S. Provisional Application No. 61/153,676, filed on February 19, 2009, the entire contents of which are incorporated herein by reference.

### BACKGROUND

### Technical Field

The present disclosure relates to a method for repairing rotator cuff injuries. More particularly, the present disclosure relates to a method for repairing rotator cuff injuries by utilizing a bone-tendon graft.

### Description of Related Art

Rotator cuff injuries, known generally as rotator cuff tears (result from the tendon damage and degeneration causing the tendon to fray and) often lead to rotator cuff tendon and/or bone-tendon separation. Fraying causes the tendon to become thinner and may result in a tendon being pulled loose from the bone. When this occurs, the tendon becomes less organized, shortened, and less robust. In most cases, where the tendon has detached from the bone, muscle atrophy and fatty degeneration occurs. This detachment, of course, causes pain and loss of function in the extensor and rotation mechanisms in a shoulder.

When rotator cuff tendons become torn they often become shortened due to forces applied by the associated muscle. This results in a gap between the normal insertion site onto the bone and the tendon. In a common surgical procedure, the shortened tendon is attempted to be pulled back into an anatomical position and secured to the humerus using a securing mechanism, e.g., suture anchors. This often requires manipulation of the tendon to gain length, however, often complete reapposition without undue tension can be unsuccessful.

After a surgical procedure has been completed, if a tendon is under a great amount of tension, any movement of the shoulder may result to an excessive force on the repair of the tendon, which may lead to shoulder pain and/or re-tear. The shortened tendon creates a medical problem that has not been effectively solved, as evidenced by the high rate of tendon re-tears known to occur after a surgical procedure to repair shoulder extensor mechanisms. In the event that the tendon cannot be reapposed to the humerus and the cable-like extensor mechanisms cannot be re-established, a graft may be used to remedy these issues.

The graft may facilitate the repair construct by one or more of the following mechanisms: i) by providing a 'bridge' to an intercalary structure between the rotator cuff tendon and the humerus in instances where shortening of the rotator cuff tendon results in a gap; ii) by augmenting the repair construct by adding additional support when the rotator cuff tendon is insufficient to handle the loads the repair construct creates; iii) by acting as a matrix for tissue repair to enhance the biological repair and reformation of the rotator cuff biomechanical integrity; and iv) by aiding recreation of the normal rotator cuff cable-like mechanism required for normal range of motion and strength.

Approximately 380,000 rotator cuff procedures are performed annually in the United States. Of these rotator cuff procedures, approximately 90,000 are massive tears of the rotator cuff, which require extensive and complex surgery. In many cases, there is a 50 percent re-tear rate after a rotator cuff surgical procedure has been performed.

A major drawback to the success of a rotator cuff repair procedure is the extended period of time required for a tendon to heal to a bone. Bone tends to heal to bone more rapidly than tendon heals to bone and the reduced time to healing is believed to influence the probability of success of repair as the mechanical fixation means, such as, sutures, hold the tendon tend to be the weak link in the repair construct. This is reflected in the typical long duration required for rehabilitation after surgery and the danger of re-tear if the construct is loaded prematurely. The bone-tendon construct also has the potential to enhance the foot-print described as the interface between the tendon and the bone, where biological healing and reattachment is crucial. Having a strong tendinous construct allows for reliance on graft strength during the healing period and also decreases the need for stretching the native tendon to acquire sufficient interface onto the humerus for reattachment.

### SUMMARY

A method of repairing a rotator cuff is disclosed. The method includes the step of accessing a surgical site including a humerus and a rotator cuff tendon, for example, via an access port.

The rotator cuff tendon may be for example, a supraspinatus tendon, an infraspinatus tendon, a teres minor tendon, a subscapularis tendon, and/or a long head tendon. In some embodiments, the rotator cuff tendon is debrided and a defect in the humerus is created. The defect may be positioned in a generally tangential to a radius of curvature of the outer surface of the humerus or generally perpendicular to a longitudinal axis of the humerus. In some embodiments, a foot-print is prepared in the humerus and/or a decortication of an outer cortex of the humerus is performed.

Furthermore, a bone-tendon assembly having a graft tendon and a bone segment is provided. The bone segment of the bone-tendon assembly may be attached to one end of a graft tendon. The method also discloses attaching the bone segment (e.g., a bone plug) of the bone-tendon assembly within the defect, such that the graft tendon extends from the humerus. The shape of the bone plug may be, for example, elliptical, cylindrical, rectangular, polygonal, or triangular. The bone-tendon assembly may include one or more bone segments and/or one or more graft tendons. In some embodiments, the bone segment may be attached to the humerus by positioning one or more dehydrated bone segments within the defect of the humerus and rehydrating the dehydrated bone segments within the defect.

Additionally, the graft tendon may be attached to the rotator cuff tendon, for example, by anchoring, suturing, adhering, screwing, stapling, plugging, or press-fitting. In some embodiments, the method may also include a step of shaping the bone defect and the bone segment to provide an interference fit between a surface of the bone segment and a surface of the bone defect when the bone segment is inserted into the bone defect. The method may also include the step of shaping the bone segment of the bone-tendon assembly to match a contour of the humerus. In other embodiments, the method of repairing a rotator cuff may further include applying energy to the surgical site with ultrasonic energy, pulsed electromagnetic field energy, current energy, and/or pressure-hyperberic energy.

In some embodiments, the graft tendon and bone segment of the bone tendon assembly may each be made of autogenous material, allogeneic material, xenogeneic material, and/or synthetic material. In addition, the graft tendon and the bone segment of the bone tendon assembly may be coupled to each other by alternative connecting means, for example, interference fitting, pinning, stapling, adhering or other mechanical means.

### BRIEF DESCRIPTION OF THE DRAWINGS

Various embodiments of the presently disclosed bone-tendon and its method of use are disclosed herein with reference to the drawings, wherein:
Fig. 1 is a side cross-sectional view of one embodiment of the presently disclosed bone-tendon assembly having a plurality of bone plugs and a graft tendon attached to a rotator cuff tendon and a humerus;
Fig. 2 is a perspective view of the bone-tendon assembly of FIG. 1 having a single bone plug and a graft tendon;
Fig. 3 is a side cross-sectional view of another embodiment of the bone-tendon assembly having a suture anchor and a bone plug attached to a rotator cuff tendon and a humerus;
Fig. 4 is a side cross-sectional view of yet another embodiment of the bone-tendon assembly having a bone screw and a bone plug attached to a rotator cuff tendon and a humerus; and
Fig. 5 is side cross-sectional view of still yet another embodiment of the bone-tendon assembly having a bone screw and a plurality of sutures attached to a rotator cuff tendon and a humerus.

### DETAILED DESCRIPTION

The attached figures illustrate exemplary embodiments of the present disclosure and are referenced to describe the embodiments depicted therein. Hereinafter, the disclosure will be described in detail by explaining the figures wherein like reference numerals represent like parts throughout the several views.

The present disclosure provides a method for repairing a torn or detached rotator cuff tendon by reattaching the same to the humeral head of the humerus via a bone-tendon graft assembly. The bone-tendon assembly generally includes a graft tendon having one or more bone segments attached thereto. The disclosed method achieves bone-to-bone fixation on the humerus, while achieving a tendon-to-tendon fixation on the rotator cuff tendon.

### Bone-Tendon Graft Assembly

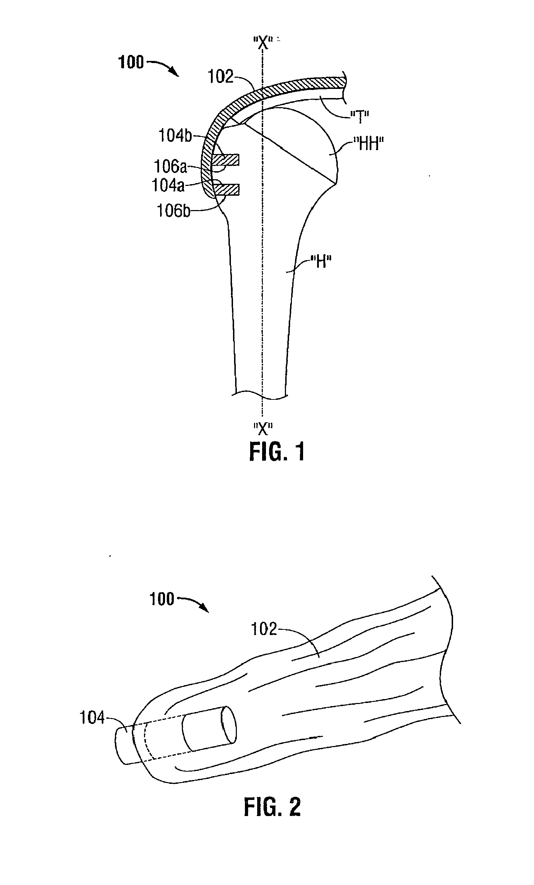

Referring now to Fig. 1, a bone-tendon assembly is illustrated for use with a method for repairing a rotator cuff tendon and is generally depicted as numeral 100. The bone-tendon assembly 100 includes a graft tendon 102 and one or more bone segments 104a and 104b. The graft tendon 102 is adapted to attach to a rotator cuff tendon T by any suitable attaching technique, for example, but not limited to, suturing, anchoring, or gluing. In this illustration, the bone-tendon assembly 100 includes two bone segments 104a and 104b (e.g., a bone plug) which are adapted to be received within a prepared host bed or bony defect formed in a humeral head HH of a humerus H. Each of bone plugs, 104a and 104b, is securely fastened to a respective bone defect 106a and 106b (e.g., hole or cavity). The bone defects 106a and 106b are created in the bony surface of the top portion of the humerus H to allow bone segments 104a and 104b to be secured therewithin. A more detailed explanation of a bone defect and its preparation will be described in further detail below.

Turning now to Fig. 2, the bone-tendon assembly 100 is shown before attachment to the rotator cuff tendon T. It is envisioned that the bone-tendon assembly 100 may be about 1 mm thick, about 6-8cm long, and the graft tendon 102 may taper from about 2-5 cm, i.e., where the graft tendon 102 attaches to the rotator cuff tendon T, to about 1-2cm, i.e., near the bone segment 104. Further, bone segment 104 may be about lcm long and about 3-6mm in diameter. The bone segments of the present disclosure may be made from cortical, cancellous bone segments, or both, which may be obtained from allogenic, autogenic, and/or xenogenic sources.

In embodiments, bone-tendon assembly 100 of the present disclosure may be constructed from a xenograft (i.e., a graft from an animal other than a human), an allograft (i.e., a graft from another human or cadaver), and/or an autograft (e.g., a graft from the same human) and/or an appropriate synthetic or combinations of the above. The bone-tendon assembly 100 may be harvested from a pes anserinus tendon complex, a gracilis muscle, or any other compatible muscle. The pes anserinus graft may be removed from a tibial insertion point on a proximal/medial location of a tibia. It is important to note that the bone segment 104 of the harvested graft, along with the attached tendon 102, may be kept intact and not separated. In this manner, the bone-tendon assembly 100 maintains a naturally, strong connection between the graft tendon 102 and the bone segment 104. Alternatively, the bone and tendon may be harvested or prepared separately and subsequently attached to one another by appropriate means including interference fit, pinning, gluing or other mechanical means. Also depicted in Fig. 2, the bone segment 104 may be cut and carved into a cylindrical shape, e.g., 3-6 mm in diameter, such that, it may be received by a conforming defect (i.e., 3-6 mm in diameter) in the humerus H of a patient. Alternative shapes are also contemplated, the receiving aperture in the humerus would be created to receive or correspond to the shape of the bone-tendon segment.

Also depicted in Figure 2 is an exemplary embodiment where the bone-tendon graft is provided with sutures attached to facilitate the implant procedure. This approach provides the benefit of reducing operating room time, and also allows for a more sophisticated attachment of the sutures to the graft to improve the biomechanical characteristics and prevent potential failure modes such as the suture cutting through the graft. Alternative or additional pre-prepared securement means are also contemplated to be integral to the bone-tendon graft, including suture anchors, or other mechanical securement devices. Alternatively the bone-tendon graft may include securement means such as biologically compatible adhesives, or other non-traditional means of establishing the repair construct.

In other embodiments, the bone-tendon assembly 100 may be constructed from a xenograft of a porcine tibia. It may be beneficial to utilize porcine-derived xenografts, since they are not associated with prion-, bse-, or scrapie-type communicable diseases. Porcine xenografts may be processed by methods such as those used by Tissue Science Laboratories, LLC (TSL). TSL uses a system for preparing porcine dermis which may be used for hernia and rotator cuff repairs. These methods use enzymatic digestion (e.g., trypsin) to remove the immunogenic domains of collagen, to aid in decellularization, and to remove attached glyoproteinacous materials (e.g., cell-surface antigens, e.g. α-GAL). It is also known that acetone may be utilized to defat and decellularize the tissue for further reducing antigenicity. An additional method disclosed by TSL is a processing method that utilizes agents to crosslink tissue. This results in a material that has limited degradation and reduced immunological potential such that there is a high level of biocompatibility. The extent of cross-linking or biological stabilization of the bone-tendon assembly can be controlled in order to achieve the appropriate balance between preventing a very high resorption rate and a very low biological incorporation and remodeling rate. Cross-linking also has the capability of enhancing biomechanical properties as it creates chemical bonds between collagen fibrils.

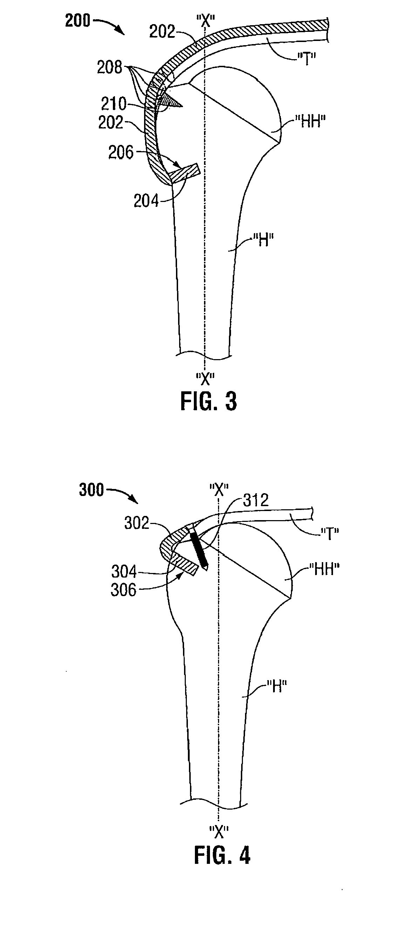

The embodiments of Figs. 3-5 are similar with respect to the described bone-tendon assembly of Fig. 1 and will only be described herein to the extent necessary to describe the differences between the embodiments.

Turning now to Fig. 3, another exemplary embodiment of a bone-tendon assembly 200 is illustrated. In this embodiment, bone-tendon assembly 200 joins the rotator cuff tendon T and the top portion of the humerus H by utilizing a graft tendon 202, a bone plug 204, a plurality of sutures 208, and a suture anchor 210. It is envisioned that the bone-tendon assembly 200 may be attached, to the top portion of the humerus H and the rotator cuff tendon T by any number of bone plugs 204 and suture anchors 210. The suture anchors 210 are inserted and secured into the humeral head HH in a screw-type or an interference-type manner. The suture anchor includes an eyelet or other engagement structure to allow a suture to pass therethrough. In this manner, the suture is secured to the anchor. The suture anchors and the sutures may be made of non-absorbable or bioabsorbable material. The sutures 208 secure graft tendon 202 to the rotator cuff tendon T. The bone plug 204 is configured to be inserted into a pre-drilled defect 206 in the humerus H, thereby creating a strong and secure bone-to-bone connection. In this configuration, the rotator cuff tendon T is securely attached to the humerus H via the bone-graft assembly 200.

Fig. 4 illustrates another embodiment of a bone-tendon assembly 300 having a graft tendon 302, a bone plug 304, and a bone screw 312. The bone plug 304 is attached to the top portion of the humerus H in a similar manner as mentioned in the embodiments above. The bone screw 312, which may be bioabsorbable or non-absorbable, is configured to pass through both, graft tendon 302 and rotator cuff tendon T, to secure graft tendon 302 to rotator cuff tendon T. In this manner, the rotator cuff tendon T is attached to the bone-tendon assembly 300 by the bone screw 312, while the bone plug 304 attaches the bone-tendon assembly 300 to the humerus H. Thus, the humerus H is securely attached to the rotator cuff tendon T via the bone-tendon assembly 300.

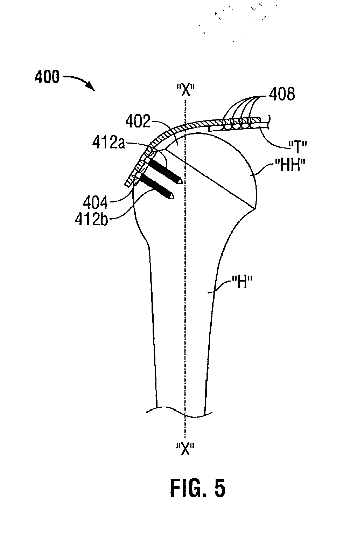

Fig. 5 illustrates a bone-tendon assembly 400 having a bone segment 404, a graft tendon 402, one or more bone screws 412a and 412b, and a plurality of sutures 408. To facilitate securement of the bone-tendon assembly 400 to the humerus H, the bone screws 412a and 412b are passed through the graft tendon 402 and the bone segment 404 and into the humerus H. The bone-tendon assembly 400 is also secured to the graft tendon 402 by sutures 408. In this manner, the rotator cuff tendon T is securely attached to the humerus H via the bone-tendon assembly 400.

### Method for Repairing a Rotator Cuff Tear

A method of repairing a rotator cuff in a patient is further disclosed in the present disclosure. It should be noted that the method of repairing a rotator cuff in the present disclosure may be utilized with any one of the embodiments discussed above, namely, bone-tendon assemblies 100, 200, 300, and 400. Further, other bone-tendon assemblies, not disclosed in the present disclosure, may be utilized by the method discussed below. For purposes of brevity, only bone-tendon assembly 100 will be described in the method described below. It should also be noted that the methods described in the present disclosure may apply to the repair of other tendons in the human body. It should also be noted that this method of the present disclosure may be applied to repair bone-tendon mechanisms of animals other than humans.

The method includes accessing a surgical site, including a humerus H and a rotator cuff tendon T. The rotator cuff tendon T may be for example, a supraspinatus tendon, an infraspinatus tendon, a teres minor tendon, a subscapularis tendon, and/or a long head tendon. Other potential tendons in various anatomical locations are also contemplated. The surgical site is accessed by performing traditional open surgery, arthroscopic surgery, and/or a mini-open surgery.

After the surgical site has been accessed, the rotator cuff tendon T is then debrided, in order to remove frayed intra-substance tissue from the torn tendon. Afterwards, the rotator cuff tendon T is pulled back into anatomical position and secured to the graft tendon 102 by using attaching means, for example, but not limited to, sutures, suture anchors, etc. In some instances the tendon may have shortened due to degeneration and contracture and may not be able to be reapposed to the anatomical insertion point without creating undue tension. In these instances, the bone-tendon assembly 100 can act as an intercalary 'bridge' to span the gap. In other instances an open 'window' remains after the repair and it is desirable to close the hole which may be responsible for residual pain in some patients.

The method of the present disclosure provides the capability of achieving both of these objectives, since the strong biomechanical properties of the disclosed embodiments protect the extensor mechanisms and facilitate natural healing. After a damaged rotator cuff tendon T (e.g., a supraspinatus tendon) has been debrided, a foot-print in the top portion of the humerus H is prepared by performing a light decortication. This foot-print enhances biological incorporation and reattachment of the rotator cuff tendon T and/or bone-tendon assembly 100 to the humerus H, thus recreating a natural-like insertion site. A defect is also created in the humerus with a configuration appropriate to correspond to the shape of the bone plug or bone plugs, e.g., 104a and 104b, of the bone tendon assembly 102.

Once the foot-print is prepared, a drilling instrument, or any other defect creating device, may be used to create the defect (e.g., hole or cavity) in humerus H. In embodiments, a diameter of the defect or defects, e.g., 106a and 106b, is dimensioned to be equal or slightly smaller than a diameter of the bone plug or plugs, e.g., 104a and 104b, such that, a compression and/or interference fit is created when the bone plug is firmly positioned within the defect. It is also anticipated that additional securement means may be utilized including mechanical means such as interference screws, etc, or other means such as adhesives, etc.

After the bone defect or defects, e.g., 106a and 106b, have been created, the bone segment or segments, e.g., 104a and 104b, are secured within the bone defects by any suitable press-fitting technique. The bone segments of the bone-tendon assembly 100 may be shaped or configured to be bone plugs. In embodiments, the shape of the bone segments may be for example, but not limited to, elliptical, rectangular, polygonal, cylindrical, or triangular.

The bone to bone fit of a surface of the bone segment or segments within a surface, e.g., a wall, of the bone defect or defects creates an interference fit to minimize irritation at the operation site when the bone segment is inserted into the bone defect. Since the presently disclosed bone-tendon assemblies may be made of autografts, allografts, and/or xenografts, the natural shape of the bone segment or segments of the bone-tendon assembly may not match the contours of the humeral head HH of the humerus H and/or the bone defect. Therefore, accurate measurements and preparation, e.g., shaping of the bone segment of the bone graft assembly 100 and the bone defect are taken in order to avoid potential complications (e.g., rubbing of surrounding tissues). This requires the length, width, and depth measurements of the bone segment of the bone-tendon assembly 100 to match the measurements of the bone defect of the prepared humerus H. Further, the contour of the bone-tendon assembly 100 may also be matched to the contour of the humeral head HH of the humerus H.

The bone segment of the bone-tendon assembly 100 is attached to the rotator cuff tendon T by an attaching technique, for example, but not limited to, anchoring, suturing, adhering with a bioadhesive, screwing, plugging, or press-fitting. A useful and beneficial feature of the presently disclosed bone-tendon assembly 100 is the ability to accurately measure the length of the graft tendon needed to avoid over-tensioning of the rotator cuff tendon T or any other native tendons, when the bone-tendon assembly is anatomically attached.

Next, the graft tendon 102 is attached to the rotator cuff tendon T by different attaching means, for example, but not limited to, clips, sutures, barbed sutures, bioadhesives, and/or any combinations thereof.

In embodiments, one or more dehydrated (e.g., lyophilized) bone segments 104 may be utilized in conjunction with any of the aforementioned embodiments discussed above. When the bone segments, e.g., 104a and 104b, are dehydrated or lyophilized, this results in shrinkage of the bone segments. In this configuration, dehydrated bone segments 104a and 104b may be positioned within the pre-drilled and/or shaped defects of humerus H, whereupon rehydration of bone segments will hydrate and expand to fill the defects, thus creating a tight, compressed fit between the bone plug and the defect.

In embodiments, additional biologically active materials may be added to enhance healing of the bone-tendon assembly to the humerus H and the tendon T, which may include growth factors, demineralized bone matrix, cells, genes, peptides, drugs (including polymer drugs) growth factors (Bone Morphogenic Proteins such as BMP-2, 4, 7, 12, or 14; Platelet Derived Growth Factors e.g. PDGF-β; Insulin-Like Growth Factors, Fibroblast Growth Factors, or other appropriate growth factors), cells (autogenous, allogenic or xenogeneic fibroblasts, muscle, fat, mesenchymal stem cells, or other appropriate cells) or other agents which may facilitate the healing process. These biologically active materials may be combined with any of the devices and materials utilized in the present disclosure including, but not limited to, tendons, sutures, adhesives, etc. Furthermore, these biologically active materials may be applied in situ as a solution or spray.

In other embodiments, the one or more defects may be positioned at a location generally perpendicular to a longitudinal axis X of the humerus, which is depicted in Figs. 1 and 3-5. In other embodiments, the defect may be positioned at a location generally tangential to a radius of curvature of the outer surface of the humeral head HH of the humerus H.

In addition, the thickness of the graft tendon can be matched to be appropriate for a particular individual such that the construct does not become too bulky and create rubbing/impingement on anatomical structures. The thickness is typically about 1-2mm.

In experiments, benchmark biomechanical properties for tendon repair products have shown 15% ultimate strain, approximately 15 mpa ultimate stress, 500-1000N ultimate load, approximately 150 mpa modulus, and 75-150 n/mm stiffness. These parameters are significantly lower the native rotator cuff tendons, indicating the need for a more biomechanically appropriate assembly as disclosed in the embodiments. Thus, initial stiffness is a key parameter in order to support the construct and protect the native tendon during the healing process.

In embodiments, energy may be applied to the surgical site, including for example, but not limited to, ultrasonic energy, pulsed electromagnetic field energy, current energy, and/or pressure-hyperbaric energy. It is known that applying energy to a surgical site, especially during or after a surgical operation, promotes rapid and effective healing.

Although the illustrative embodiments of the present disclosure have been described herein with reference to the accompanying drawings, it is to be understood that the disclosure is not limited to those precise embodiments and that various other changes and modifications may be effect therein by one skilled in the art without departing from the scope or spirit of the disclosure.

## Claims

1. Use of at least one graft tendon and at least one bone tendon (bone-tendon assembly) in the preparation of a medicament for the repair of a rotator cuff, comprising the steps of:
accessing a surgical site including a humerus and a rotator cuff tendon;
creating a defect in the humerus;
providing the bone-tendon assembly including at least one graft tendon and at least one bone segment;
attaching the at least one bone segment of the bone-tendon assembly within the defect of the humerus such that the at least one graft tendon extends from the humerus; and
attaching the at least one graft tendon to the rotator cuff tendon.

2. The use according to claim 1 further comprising the step of:
debriding the rotator cuff tendon.

3. The use according to claim 1 or claim 2, wherein the step of creating a defect in the humerus includes preparing a foot-print in the humerus.

4. The use according to claim 3, wherein the step of preparing a footprint includes performing a decortication of an outer cortex of the humerus.

5. The use according to any preceding claim, wherein the step of providing a bone-tendon assembly includes attaching the bone segment to one end of the graft tendon.

6. The use according to any preceding claim, wherein the step of accessing the humerus and the rotator cuff includes accessing the humerus and the rotator cuff via an access port.

7. The use according to any preceding claim, wherein the rotator cuff tendon is selected from the group consisting of a supraspinatus tendon, an infraspinatus tendon, a teres minor tendon, a subscapularis tendon, and a long head tendon.

8. The use according to any preceding claim, wherein the bone segment of the bone-tendon assembly is a bone plug, preferably wherein the shape of the bone plug is selected from the group consisting of elliptical, cylindrical, rectangular, polygonal, and triangular.

9. The use according to any preceding claim, wherein the at least one bone segment of the bone-tendon assembly includes a plurality of bone segments and/or the use according to any preceding claim, wherein the at least one graft tendon of the bone-tendon assembly includes a plurality of graft tendons.

10. The use according to any preceding claim, wherein the at least one graft tendon and the at least one bone segment of the bone-tendon assembly includes a plurality of graft tendons and a plurality of bone segments.

11. The use according to any preceding claim, wherein the step of attaching the graft tendon of the bone-tendon assembly to the rotator cuff tendon is performed by an attaching technique selected from the group consisting of anchoring, suturing, adhering, stapling, screwing, plugging, and press-fitting and/or the use according to any preceding claim, wherein the step of attaching the at least one bone segment further includes positioning at least one dehydrated bone segment within the defect of the humerus and rehydrating the at least one dehydrated bone segment.

12. The use according to any preceding claim, further comprising the step of:
applying energy to the surgical site selected from the group consisting of ultrasonic energy, pulsed electromagnetic field energy, current energy, and pressure hyperbaric energy.

13. The use according to any preceding claim, wherein the step of creating the defect in the humerus further includes positioning the defect at a position generally perpendicular to a longitudinal axis of the humerus or the use according to any preceding claim, wherein the step of creating the defect in the humerus further includes positioning the defect at a position generally tangential to a radius of curvature of the outer surface of the humerus.

14. The use according to any preceding claim further comprising the step of:
shaping the at least one bone segment and the at least one bone defect to provide a interference fit between a surface of the at least one bone segment and a surface of the at least one bone defect when the at least one bone segment is inserted into the at least one bone defect and/or the use according to any preceding claim further comprising the step of:
shaping the at least one bone segment of the bone-tendon assembly to match a contour of the humerus.

15. The use according to any preceding claim, wherein the at least one graft tendon and the at least one bone segment of the bone tendon assembly is each comprises a material selected from the group consisting of autogenous, allogenic, xenogenic and synthetic.

16. The use according to any preceding claim, wherein the at least one graft tendon and the at least one bone segment of the bone tendon assembly are coupled to each other by using mechanical means selected from the group consisting of interference fitting, pinning, stapling, adhering and combinations thereof.
